# EUROPEAN PATENT APPLICATION

(11) **EP 4 425 183 A1**
(43) Date of publication of application: **04.09.2024**
(21) Application number: 22887189.3
(22) Date of filing: 28.10.2022
(51) Int. Cl.: G01N 33/86, C12Q 1/37, G01N 33/48

(54) **METHOD FOR EVALUATING FIBRINOLYSIS RESISTANCE ACTIVITY IN BLOOD PLASMA**

(30) Priority: 29.10.2021 JP 2021177098
(71) Applicant: National University Corporation Hamamatsu University School of Medicine, Hamamatsu-shi, Shizuoka 431-3192 (JP)
(72) Inventor: SUZUKI Yuko, Hamamatsu-shi, Shizuoka 431-3192 (JP); URANO Tetsumei, Hamamatsu-shi, Shizuoka 431-3192 (JP)
(74) Representative: LLR
(86) International application number: PCT/JP2022/040448
(87) International publication number: WO 2023/074864

(57) **Abstract**

An object of the present invention is to provide a method for easily evaluating residual fibrinolytic resistance activity by PAI-1 and α2AP in blood plasma. The present invention is a method of evaluating fibrinolytic resistance activity, the method including a pretreatment step of adding an anionic surfactant to some of a blood plasma sample derived from a test animal and incubating the blood plasma for a predetermined time, a first fibrinolytic resistance measurement step of measuring an index value of fibrinolytic resistance activity in treated blood plasma obtained by the pretreatment step, a second fibrinolytic resistance measurement step of measuring an index value of fibrinolytic resistance activity in some of the blood plasma sample derived from the test animal, and an evaluation step of evaluating the fibrinolytic resistance activity by PAI-1 and α2AP in the blood plasma of the test animal, based on the index value measured in the first fibrinolytic resistance measurement step and the index value of untreated blood plasma measured in the second fibrinolytic resistance measurement step.

## Description

### [Technical Field]

The present invention relates to a method for easily evaluating residual fibrinolytic resistance activity of PAI-1 and α2AP in blood plasma.

Priority is claimed on Japanese Patent Application No. 2021-177098, filed October 29, 2021, the content of which is incorporated herein by reference.

### [Background Art]

Thrombolytic therapy is used for the treatment of thrombosis such as cerebral infarction. The treatment method is a treatment method in which plasminogen activators (PAs) such as tissue plasminogen activator (tPA) activate plasminogen in blood or on the surface of a thrombus, and the generated plasmin lyses fibrin, which is a major component of the thrombus. In order to improve the symptoms and avoid sequelae, rapid thrombolysis is required.

Anti-fibrinolytic therapy is used for the treatment of hemorrhagic pathologies caused by hyperfibrinolysis. The treatment method is a treatment method in which the binding of plasminogen or plasmin to the surface of a thrombus is inhibited by tranexamic acid to suppress the activation of plasminogen on fibrin and the fibrinolysis by plasmin. That is, in the anti-fibrinolytic therapy, the bleeding symptoms can be improved by suppressing excessive fibrinolytic activity.

In blood plasma, plasminogen activator inhibitor type 1 (PAI-1) and α2-antiplasmin (α2AP), which are a physiological inhibitor of PAs and plasmin, are present and control the expression of excessive fibrinolytic (fibrinogen lysis) activity. The fibrinolytic activity expression ability of blood is determined by the balance between the amount of tPA and the amount of PAI-1 in blood plasma (see Non Patent Documents 1 and 2). It is necessary to administer PAs, which exceeds the amount of PAI-1, for rapid production of plasmin and it is necessary to produce plasmin in an amount that consumes a certain amount of α2AP in blood plasma for efficient thrombolysis. On the other hand, in the case where PAs are administered in an amount such that the α2AP amount in blood plasma is excessively consumed, not only fibrin but also fibrinogen in the blood plasma are degraded by plasmin, and systemic uncontrolled bleeding is exhibited. Therefore, in the thrombolytic therapy, it is important to frequently quantify the residual amount of PAI-1 and the residual amount of α2AP in the blood plasma for determining a safe and efficient dose of PAs. In addition, in a hyperfibrinolytic state such as after a head trauma, it is important to quantify the residual amount of PAI-1 and the residual amount of α2AP in the blood plasma to confirm the consumption thereof and to grasp the hyperfibrinolytic pathophysiology for determining a propriety of an anti-fibrinolytic therapy using tranexamic acid can be performed. In the case where the fibrinolytic resistance is attenuated because of the consumption of PAI-1 and α2AP in the blood plasma, it is necessary to administer tranexamic acid. On the other hand, in the case where the PAI-1 as an acute phase protein is increased and the fibrinolytic resistance is enhanced, the risk of organ damage associated with the formation of a microthrombus is increased. Therefore, the administration of tranexamic acid should be avoided.

At present, a specific measurement kit is required to measure the activity level or the antigen amount of the residual PAI-1 and the residual α2AP in the blood plasma, and in the case where the measurement kit is used, a long time is required to obtain the result. As a method for measuring coagulation and fibrinolytic activity that can be operated at the bedside, there is a method for measuring coagulation and fibrinolytic activity using a change in viscosity of a blood clot as an index, such as thromboelastography (TEG) and rotational thromboelastometry (ROTEM), but the residual activities of PAI-1 and α2AP in blood plasma cannot be measured by these methods.

As described above, in the current thrombolytic therapy for acute cerebral infarction and the like, the quantification of fibrinolytic resistance is not performed, and a method of administering PAs in accordance with a protocol that emphasizes safety is recommended. However, in patients with sepsis or thrombosis associated with COVID-19 (novel coronavirus infection that occurred in 2019), the concentrations in blood of fibrinolysis factors such as plasminogen, fibrinogen, PAI-1, and α2AP are often deviated from the normal values, and in a standard protocol, sufficient therapeutic efficacy may not be obtained or there is a possibility of excessive administration. tPA treatment of severe patients with COVID-19 has also been attempted, but the problem is that it is difficult to set the dose. In addition, hemorrhagic pathologies that cause hyperfibrinolysis are observed in the hyperacute stage of multiple traumas and head traumas, the perinatal stage, and the like. In particular, in the former, since the pathologies are transferred to fibrinolysis blockade in the subacute stage, it is necessary to quantify fibrinolytic resistance and to grasp attenuation as a determination of a propriety an anti-fibrinolytic therapy can be performed and the appropriate timing of adaptation.

On the other hand, it has been reported by the inventors that there are three forms of PAI-1, an active form that forms a co-complex with PAs, an active latent form that does not react with PAs, and a substrate form that is easily cleaved by PAs, and that the active form PAI-1 loses its inhibitory activity by structural changes to the active latent form or the substrate form in the case where it is treated with 0.1% or 0.01% sodium dodecyl sulfate (SDS), and in the case where it is treated with Triton X-100, the inhibitory activity is not affected even in the presence of PAI-1, and the activity of PAs can be sufficiently measured (Non Patent Document 3). It has also been reported that α2AP is also inactivated by treatment with 0.1% or 0.01% SDS, and plasmin activity can be sufficiently measured even in the presence of α2AP together with Triton X-100 (Non Patent Document 4).

### [Citation List]

### [Non Patent Documents]

[Non Patent Document 1]
   Urano, et al., Thrombosis and Haemostasis, 1990, vol. 63, p. 82-86.
[Non Patent Document 2]
   Urano, et al., Thrombosis and Haemostasis, 1991, vol. 66, p. 474-478.
[Non Patent Document 3]
   Urano, et al., European Journal of Biochemistry, 1992, vol. 209, p. 985-992.
[Non Patent Document 4]
   Dong, et al., Thrombosis Research, 1993, vol. 69, p.491-499.

### [Summary of Invention]

### [Technical Problem]

In the thrombolytic therapy, in order to determine a dose of PAs that is safe and can be expected to have sufficient thrombolysis even in patients in whom the concentration of a fibrinolysis factor in blood deviates from a normal value, a method for easily measuring the fibrinolytic resistance activity of PAI-1 and α2AP in blood plasma of each patient is required. In addition, in the anti-fibrinolytic therapy, it is also important to measure the fibrinolytic resistance activity of PAI-1 and α2AP in the blood plasma also for appropriately grasp the hyperfibrinolytic pathophysiology by grasping the reduced consumability of the fibrinolysis inhibitor. Therefore, an object of the present invention is to provide a method for easily evaluating residual fibrinolytic resistance activity by PAI-1 and α2AP in blood plasma.

### [Solution to Problem]

The inventors of the present invention have found that by adding tPA to blood plasma treated with SDS and lysing the plasma clot in the presence of Triton X-100, even in the case where PAI-1 and α2AP are present, it is possible to measure the PA-dependent plasma clot lysis time and to measure the plasmin generation rate by a fluorescent substrate without being affected by the activities of the PAI-1 and the α2AP, and have completed the present invention.

That is, the present invention is as follows.
[1] A method of evaluating fibrinolytic resistance activity in blood plasma of a test animal, the method comprising:
   a pretreatment step of adding an anionic surfactant to some of a blood plasma sample derived from the test animal and incubating the blood plasma sample for a predetermined time;
   a first fibrinolytic resistance measurement step of measuring an index value of fibrinolytic resistance activity by PAI-1 and α2AP in the treated blood plasma sample obtained by the pretreatment step;
   a second fibrinolytic resistance measurement step of measuring an index value of fibrinolytic resistance activity by PAI-1 and α2AP in some of the blood plasma sample derived from the test animal; and
   an evaluation step of evaluating the fibrinolytic resistance activity by PAI-1 and α2AP in the blood plasma of the test animal, based on the index value measured in the first fibrinolytic resistance measurement step and the index value of untreated blood plasma measured in the second fibrinolytic resistance measurement step,
   wherein the index value is the time until a plasma clot formed by thrombin in the presence of a nonionic surfactant and a plasminogen activator is lysed, or a plasmin generation rate in the case of adding thrombin in the presence of a nonionic surfactant and a plasminogen activator.
[2] The method of evaluating fibrinolytic resistance activity in blood plasma according to [1],
   wherein the anionic surfactant is SDS.
[3] The method of evaluating fibrinolytic resistance activity in blood plasma according to [2],
   wherein the pretreatment step is performed by adding 0.01 to 0.1 w/v% of SDS to some of the blood plasma sample and treating the blood plasma sample for 5 to 30 minutes.
[4] The method of evaluating fibrinolytic resistance activity in blood plasma according to any one of [1] to [3],
   wherein the nonionic surfactant is Triton X-100.
[5] The method of evaluating fibrinolytic resistance activity in blood plasma according to any one of [1] to [4],
   wherein the index value is the time until the plasma clot formed by thrombin in the presence of the nonionic surfactant and the plasminogen activator is lysed,
   the first fibrinolytic resistance measurement step, the second fibrinolytic resistance measurement step, and the evaluation step are each
   a first lysis time measurement step of adding a nonionic surfactant and a plasminogen activator to the treated blood plasma sample obtained in the pretreatment step, further adding thrombin thereto to form a plasma clot, and then measuring the time until the formed plasma clot is lysed from the time at which the thrombin is added,
   a second lysis time measurement step of adding a nonionic surfactant and a plasminogen activator to some of the blood plasma sample derived from the test animal, further adding thrombin thereto to form a plasma clot, and then measuring the time until the formed plasma clot is lysed from the time at which the thrombin is added, and
   an evaluation step of evaluating the fibrinolytic resistance activity by PAI-1 and α2AP in the blood plasma of the test animal based on a difference between the lysis time of the plasma clot of the treated blood plasma measured in the first lysis time measurement step and the lysis time of the plasma clot of the untreated blood plasma measured in the second lysis time measurement step, and
   concentrations of the nonionic surfactant, the plasminogen activator, and the thrombin, which are added to the untreated blood plasma in the second lysis time measurement step, are equal to concentrations of the nonionic surfactant, the plasminogen activator, and the thrombin, which are added to the treated blood plasma in the first lysis time measurement step.
[6] The method of evaluating fibrinolytic resistance activity in blood plasma according to [5],
   wherein the nonionic surfactant is Triton X-100, and
   the formation of the plasma clot in the first lysis time measurement step and the second lysis time measurement step is performed in the presence of 1.0 v/v% Triton X-100.
[7] The method of evaluating fibrinolytic resistance activity in blood plasma according to [5] or [6],
   wherein the formation of the plasma clot in the first lysis time measurement step and the second lysis time measurement step is performed in the presence of the plasminogen activators at two or more different concentrations, and
   in the evaluation step, the fibrinolytic resistance activity by PAI-1 and α2AP in the blood plasma of the test animal is evaluated based on a difference between the lysis time of the plasma clot of the treated blood plasma measured in the first lysis time measurement step and the lysis time of the plasma clot of the untreated blood plasma measured in the second lysis time measurement step.
[8] The method of evaluating fibrinolytic resistance activity in blood plasma according to any one of [1] to
   ,
   wherein the index value is the plasmin generation rate in the case of adding thrombin in the presence of the nonionic surfactant and the plasminogen activator,
   the first fibrinolytic resistance measurement step, the second fibrinolytic resistance measurement step, and the evaluation step are each
   a first plasmin generation rate measurement step of adding a nonionic surfactant, a plasminogen activator, and a plasmin fluorescent substrate to the treated blood plasma obtained by the pretreatment step, further adding thrombin thereto to form a plasma clot, and then measuring a plasmin generation rate from the time at which the thrombin is added,
   a second plasmin generation rate measurement step of adding a nonionic surfactant, a plasminogen activator, and a plasmin fluorescent substrate to some of the blood plasma sample derived from the test animal, further adding thrombin thereto to form a plasma clot, and then measuring a plasmin generation rate from the time at which the thrombin is added, and
   an evaluation step of evaluating the fibrinolytic resistance activity by PAI-1 and α2AP in the blood plasma of the test animal based on a ratio of the plasmin generation rate in the treated blood plasma measured in the first plasmin generation rate measurement step to the plasmin generation rate in the untreated blood plasma measured in the second plasmin generation rate measurement step, and
   concentrations of the nonionic surfactant, the plasminogen activator, the plasmin fluorescent substrate, and thrombin, which are added to the untreated blood plasma in the second plasmin generation rate measurement step, are equal concentrations of the nonionic surfactant, the plasminogen activator, the plasmin fluorescent substrate, and the thrombin, which are added to the treated blood plasma in the first plasmin generation rate measurement step.
[9] The method of evaluating fibrinolytic resistance activity in blood plasma according to [8],
   wherein the plasmin generation rate is a value obtained by dividing an amount of change in a fluorescence brightness value from the point of time at which the thrombin is added by a square of an elapsed time from the point of time at which the thrombin is added.
[10] The method of evaluating fibrinolytic resistance activity in blood plasma according to [8] or [9],
   wherein the nonionic surfactant is Triton X-100, and
   the formation of the plasma clot in the first plasmin generation rate measurement step and the second plasmin generation rate measurement step is performed in the presence of 1.0 v/v% Triton X-100.
[11] A method for determining a dose of a plasminogen activator in a thrombolytic therapy, the method comprising:
   performing the method of evaluating fibrinolytic resistance activity in blood plasma according to any one of [1] to [10] on a blood plasma sample derived from a patient on whom the thrombolytic therapy is performed; and
   determining the dose of the plasminogen activator in the thrombolytic therapy based on an obtained evaluation result.
[12] A method for determining a propriety of tranexamic acid administration in an anti-fibrinolytic therapy, the method comprising:
   performing the method of evaluating fibrinolytic resistance activity in blood plasma according to any one of [1] to [10] on a blood plasma sample derived from a patient on whom the anti-fibrinolytic therapy is performed; and
   determining the propriety of tranexamic acid administration in the anti-fibrinolytic therapy based on an obtained evaluation result.

### [Advantageous Effects of Invention]

By using the method of evaluating fibrinolytic resistance activity in blood plasma according to the present invention, the fibrinolytic resistance activity by PAI-1 and α2AP in blood plasma can be easily evaluated. Therefore, by using the evaluation method, it is possible to very simply determine the optimum dose of the plasminogen activators (PAs) that can be expected to be safe and to obtain sufficient drug efficacy in the thrombolytic therapy. In addition, the evaluation method is also very useful for determining a propriety of tranexamic acid administration in the anti-fibrinolytic therapy.

### [Brief Description of Drawings]

FIG. 1 is a diagram showing the measurement results of a treated plasma clot lysis time (minutes) and an untreated plasma clot lysis time (minutes) in normal blood plasma in Example 1.
FIG. 2 is a diagram showing the measurement results of a treated plasma clot lysis time (minutes) and an untreated plasma clot lysis time (minutes) in α2AP-deficient blood plasma in Example 1.
FIG. 3 is a diagram showing the measurement results of a treated plasma clot lysis time (minutes) and an untreated plasma clot lysis time (minutes) in normal blood plasma in Example 2.
FIG. 4 is a diagram in which the correlation (o) between the difference (minutes) between the SDS-treated plasma clot lysis time and the untreated plasma clot lysis time in the normal blood plasma and the tPA concentration (nM) and the correlation (*) between the difference (minutes) between the SDS-treated plasma clot lysis time and the untreated plasma clot lysis time in α2AP-deficient blood plasma and the tPA concentration (nM) in Example 2 are each plotted.
FIG. 5 is a diagram showing the measurement results of the plasmin generation rate (dF/min²) of treated blood plasma and the plasma plasmin generation rate (dF/min²) of untreated blood plasma in normal blood plasma in Example 3.
FIG. 6 is a diagram in which the correlation (o) between the ratio of the plasmin generation rate of the treated blood plasma to the plasmin generation rate of the untreated blood plasma in normal blood plasma and the tPA concentration and the correlation (*) between the ratio of the plasmin generation rate of the treated blood plasma to the plasmin generation rate of the untreated blood plasma in α2AP-deficient blood plasma and a tPA concentration (nM) in Example 3 are plotted.

### [Description of Embodiments]

The fibrinolytic resistance of blood plasma can be evaluated using the length of time (plasma clot lysis time) required for the plasma clot formed by adding a certain amount of tPA to the blood plasma and then adding thrombin thereto to be lysed as an index. In blood plasma having a high fibrinolytic resistance, the plasma clot lysis time is long, and in blood plasma having a low fibrinolytic resistance, the plasma clot lysis time is short.

The fibrinolytic resistance of blood plasma can also be evaluated by using the plasmin generation rate in the process until the formed plasma clot is lysed from the point of time at which a certain amount of tPA is added to the blood plasma and then thrombin is added, as an index. In the blood plasma having a high fibrinolytic resistance, the plasmin generation rate is slow and the plasmin activity is low. On the contrary, in the blood plasma having low fibrinolytic resistance, the plasmin generation rate is high and the plasmin activity is high.

The method of evaluating fibrinolytic resistance activity in blood plasma according to the embodiment of the present invention is a method for measuring an index value of the fibrinolytic resistance for each of the blood plasma subjected to the inactivation treatment of PAI-1 and α2AP and the untreated blood plasma, and evaluating the fibrinolytic resistance activity based on both of the obtained measurement values. Specifically, the method of evaluating fibrinolytic resistance activity in blood plasma according to the embodiment of the present invention is a method for evaluating the fibrinolytic resisting activity by PAI-1 and α2AP in blood plasma of a test animal, in which a plasma clot lysis time or a plasmin generation rate is used as an index value, and has the following steps
a pretreatment step of adding an anionic surfactant to some of a blood plasma sample derived from the test animal and incubating the blood plasma for a predetermined time,
a first fibrinolytic resistance measurement step of measuring an index value of fibrinolytic resistance activity by PAI-1 and α2AP in treated blood plasma obtained by the pretreatment step,
a second fibrinolytic resistance measurement step of measuring an index value of fibrinolytic resistance activity by PAI-1 and α2AP in some of the blood plasma sample derived from the test animal, and
an evaluation step of evaluating the fibrinolytic resistance activity by PAI-1 and α2AP in the blood plasma of the test animal, based on the index value measured in the first fibrinolytic resistance measurement step and the index value of untreated blood plasma measured in the second fibrinolytic resistance measurement.

In the pretreatment step, by treating the blood plasma sample to be evaluated for fibrinolytic resistance activity in advance with an anionic surfactant such as SDS, the structures of PAI-1 and α2AP in the blood plasma can be changed to inactivate the PAI-1 and the α2AP (Non Patent Documents 3 and 4). Furthermore, in the first lysis time measurement step and the second lysis time measurement step, by performing the lysis of the plasma clot in the presence of Triton X-100, the activity of the residual PAI-1 and α2AP can be suppressed and the clot lysis by plasmin can be performed (Non Patent Documents 3 and 4). Therefore, in the evaluation step, the difference between the plasma clot lysis time of the treated blood plasma and the plasma clot lysis time of the untreated blood plasma can be used as an index of the fibrinolytic resistance by PAI-1 and α2AP in the blood plasma provided for the evaluation. In addition, in the evaluation step, the ratio of the plasmin generation rate of the treated blood plasma to the plasmin generation rate of the untreated blood plasma ([plasmin generation rate of treated blood plasma]/[plasmin generation rate of untreated blood plasma]) can also be used as an index of fibrinolytic resistance by PAI-1 and α2AP in the blood plasma used in the evaluation.

The blood plasma used in the pretreatment step is prepared from blood collected from a test animal to be evaluated for fibrinolytic resistance activity in blood plasma. The test animal is not particularly limited as long as the animal has a coagulation and fibrinolysis system in which the lysis of the plasma clot is inhibited by PAI-1 and α2AP. In the method of evaluating fibrinolytic resistance activity in blood plasma according to the embodiment of the present invention, the test animal is preferably a mammal, more preferably an experimental animal such as a human, a mouse, a rat, or a monkey, and a livestock or a pet such as a rabbit, a dog, a cat, a cow, a horse, or a sheep, and particularly preferably a human. The preparation of blood plasma from blood collected from a test animal can be performed by a usual method. For example, after collecting blood from a test animal in a blood collection tube containing an anticoagulant such as sodium citrate in advance, the blood collection tube can be subjected to a centrifugation treatment to precipitate blood cell components, and blood plasma in the supernatant can be collected.

Only some of the blood plasma sample prepared from the blood collected from the test animal is used in the pretreatment step. Examples of the anionic surfactant added to the blood plasma in the pretreatment step include a higher fatty acid salt, an α-sulfo fatty acid methyl ester salt, a linear alkylbenzene sulfonic acid salt, an alkyl sulfate ester salt, an alkyl ether sulfate ester salt, a (mono)alkyl phosphate ester salt, an α-olefin sulfonic acid salt, an alkane sulfonic acid salt, and the like. As the anionic surfactant used in the present invention, an alkyl sulfate salt is preferable, and SDS, which is widely used as an additive for cosmetics and pharmaceuticals, is particularly preferable from the viewpoint of having a sufficient effect.

The amount of the anionic surfactant added to the blood plasma in the pretreatment step may be any amount that is necessary and sufficient to inactivate PAI-1 and α2AP that may be contained in the blood plasma, and can be appropriately determined in consideration of the type of the anionic surfactant to be used and the like. For example, in the case where an anionic surfactant is added to blood plasma, the plasma clot lysis time is long at a low concentration, the plasma clot lysis time is decreased concentration-dependently, and the plasma clot lysis time is constant at a certain concentration or more. Therefore, the concentration of each anionic surfactant is preferably within a concentration range in which the plasma clot lysis time is constant, and is also preferably a relatively low within the concentration range such that the influence on the biological components other than PAI-1 and α2AP is not excessive.

The incubation time after adding the anionic surfactant to the blood plasma in the pretreatment step may be any time sufficient to inactivate PAI-1 and α2AP that may be contained in the blood plasma, and can be appropriately determined in consideration of the type, concentration, and the like of the anionic surfactant to be used. For example, in the case where the incubation time is too short, the inactivation is insufficient in the case where an amount of PAI-1 and α2AP in the blood plasma is large, the reproducibility of the plasma clot lysis time is reduced, and the reliability of the evaluation of the fibrinolytic resistance activity in blood plasma finally obtained is reduced. On the other hand, in the case where the incubation time is too long, the time required for evaluation to be obtained is long. By setting the incubation time in the pretreatment step to 5 to 30 minutes, preferably 10 to 30 minutes, and more preferably 10 to 15 minutes, it is possible to achieve both reliability and rapidity of evaluation. The incubation can be performed, for example, at 0°C to 37°C, and is more preferably performed at room temperature to 37°C.

In the case where the anionic surfactant added to the blood plasma is SDS, in the pretreatment step, it is preferable to add 0.01 to 0.1 w/v% of SDS to the blood plasma and incubate the blood plasma for 5 to 30 minutes, more preferably 10 to 30 minutes, at room temperature to 37°C, preferably at room temperature (temperature under measurement environment).

### <Case where plasma clot lysis time is used as index value of fibrinolytic resistance activity by PAI-1 and α2AP>

In the method of evaluating fibrinolytic resistance activity in blood plasma according to the embodiment of the present invention, in the case where the plasma clot lysis time is used as an index value of the fibrinolytic resistance activity by PAI-1 and α2AP, the first fibrinolytic resistance measurement step is the following first lysis time measurement step, and the second fibrinolytic resistance measurement step is the following second lysis time measurement step. Therefore, the evaluation step is the following evaluation step.

A first lysis time measurement step of adding a nonionic surfactant and PAs to the treated blood plasma obtained in the pretreatment step, further adding thrombin thereto to form a plasma clot, and then measuring the time until the formed plasma clot is lysed from the time at which the thrombin is added.

A second lysis time measurement step of adding a nonionic surfactant and PAs to the part of the blood plasma derived from the test animal, further adding thrombin thereto to form a plasma clot, and then measuring the time until the formed plasma clot is lysed from the time at which the thrombin is added.

An evaluation step of evaluating the fibrinolytic resistance activity by PAI-1 and α2AP in the blood plasma of the test animal based on a difference between the lysis time of the plasma clot of the treated blood plasma measured in the first lysis time measurement step and the lysis time of the plasma clot of the untreated blood plasma measured in the second lysis time measurement step.

In a first lysis time measurement step, a nonionic surfactant and PAs are added to the treated blood plasma obtained in the pretreatment step, then thrombin is further added thereto to form a plasma clot, and the plasma clot lysis time is measured. The order of addition of the nonionic surfactant and PAs to the treated blood plasma is not particularly limited, and any of them may be added first, or both may be added at the same time. By adding the nonionic surfactant before the addition of thrombin, the PAs-dependent lysis of the plasma clot formed in the treated blood plasma can be performed in the presence of the nonionic surfactant.

Examples of the nonionic surfactant added to the treated blood plasma include polyoxyethylene alkylphenyl ether, polyoxyethylene alkyl ether alkyl glycoside, polyoxyethylene fatty acid ester fatty acid alkanolamide, polyoxyethylene sorbitan fatty acid ester, sorbitan fatty acid ester, sucrose fatty acid ester, and the like. As the nonionic surfactant used in the present invention, polyoxyethylene alkylphenyl ether is preferable, and Triton X-100, which is widely used as an additive for cosmetics and pharmaceuticals, is particularly preferable from the viewpoint of having a sufficient effect.

The amount of the nonionic surfactant added to the treated blood plasma in the first lysis time measurement step may be any amount that is necessary and sufficient to suppress the activities of PAI-1 and α2AP that may be contained in the treated blood plasma, and can be appropriately determined in consideration of the type of the nonionic surfactant used and the like. For example, in the case where a nonionic surfactant is added to blood plasma, the plasma clot lysis time is long at a low concentration, the plasma clot lysis time is decreased concentration-dependently, and the plasma clot lysis time is constant at a certain concentration or more. Therefore, the concentration of each nonionic surfactant is preferably within a concentration range in which the plasma clot lysis time is constant, and is also preferably a relatively low within the concentration range such that the influence on the biological components other than PAI-1 and α2AP is not excessive. For example, in the case of Triton X-100, it is preferable to add Triton X-100 to the treated blood plasma such that formation of a plasma clot is performed in the presence of 1.0 v/v% Triton X-100.

In the first lysis time measurement step, the PAs added to the treated blood plasma are not particularly limited as long as the PAs are substances having a plasminogen activator activity, that is, a function of activating plasminogen to generate plasmin. For example, the PAs may be tPA or urokinase type plasminogen activator (uPA). As the PAs used in the present invention, tPA is more preferable. In addition, the PAs may be naturally occurring PAs purified from animal blood plasma, or may be recombinant PAs synthesized by Escherichia coli, insect cells, yeast, animal cells, or the like. In the present invention, to suppress errors between measurements and further improve the reliability of the measurements, the purified recombinant PAs are preferable, and the purified recombinant tPA is more preferable. The amount of PAs added to the treated blood plasma is not particularly limited, and can be, for example, 0.1 to 10 nM, preferably 0.5 to 10 nM, and more preferably 1.0 to 5.0 nM.

In the first lysis time measurement step, the thrombin added to the treated blood plasma is not particularly limited as long as it is a serine protease that catalyzes a reaction of converting fibrinogen into fibrin. The thrombin may be a naturally occurring thrombin purified from animal blood plasma, or may be a recombinant thrombin synthesized by Escherichia coli, insect cells, yeast, animal cells, or the like. In the present invention, to suppress an error between measurements and to further improve the reliability of the measurement, the purified recombinant thrombin is preferable. The amount of the thrombin added to the treated blood plasma is not particularly limited, and can be, for example, 0.1 to 10 units, and preferably 0.5 to 5 units.

In the case where thrombin is added to the treated blood plasma to which the nonionic surfactant and PAs have been added, plasma clots are created. The plasma clot is lysed by plasmin generated by PAs. The time until the formed plasma clot is lysed from the time at which the thrombin is added is measured as a plasma clot lysis time. The presence or absence of lysis of the plasma clot can be determined by measuring the absorbance as a change in turbidity.

Among the blood plasma derived from the test animal, the residual portion or some of the residual portion which is not used in the pretreatment step (untreated blood plasma) is used in the second lysis time measurement step. The second lysis time measurement step is performed in the same manner as the first lysis time measurement step except that untreated blood plasma is used instead of the treated blood plasma. That is, in the second lysis time measurement step, the same type of nonionic surfactant, PAs, and thrombin as those added to the treated blood plasma in the first lysis time measurement step are used. Furthermore, the concentrations of the nonionic surfactant, PAs, and thrombin added to the untreated blood plasma are equal to the concentrations of the nonionic surfactant, PAs, and thrombin added to the treated blood plasma in the first lysis time measurement step.

The plasma clot lysis time (treated plasma clot lysis time: T¹) of the treated blood plasma measured in the first lysis time measurement step is the time until the plasma clot formed by thrombin in the presence of the nonionic surfactant and the PAs is lysed. Specifically, the treated plasma clot lysis time indicates the time ([T¹ (minutes)] = [T¹_{E} (minutes)] - [T¹_{S} (minutes)]) from the point of time (T¹_{S}) at which thrombin was added to the treated blood plasma to which the nonionic surfactant and the PAs have been added to the point of time (T¹_{E}) at which the plasma clot in the treated blood plasma is lysed. Similarly, the plasma clot lysis time (untreated plasma clot lysis time: T²) of the untreated blood plasma measured in the second lysis time measurement step indicates the time ([T² (minutes)] = [T²_{E} (minutes)] - [T²_{S} (minutes)]) from the point of time (T²_{S}) at which thrombin is added to the treated blood plasma to which the nonionic surfactant and PAs have been added to the point of time (T²_{E}) at which the plasma clot in the treated blood plasma is lysed.

The difference between the lysis time of the plasma clot of the treated blood plasma measured in the first lysis time measurement step (treated plasma clot lysis time) and the lysis time of the plasma clot of the untreated blood plasma measured in the second lysis time measurement step (untreated plasma clot lysis time) is dependent on the fibrinolytic resistance by PAI-1 and α2AP in the blood plasma. Therefore, in the evaluation step, the fibrinolytic resistance activity by PAI-1 and α2AP in the blood plasma of the test animal is evaluated based on the difference between the treated plasma clot lysis time and the untreated plasma clot lysis time. It can be evaluated that the larger the difference between both plasma clot lysis times is, the higher the fibrinolytic resistance activity by PAI-1 and α2AP in the blood plasma of the test animal is, and the smaller the difference between both plasma clot lysis times is, the lower the fibrinolytic resistance activity by PAI-1 and α2AP in the blood plasma of the test animal is.

In the present invention, the fibrinolytic resistance activity by PAI-1 and α2AP in blood plasma can also be expressed by the concentration of PAs in blood plasma. The untreated plasma clot lysis time is shortened depending on the amount of the added PAs, whereas the treated plasma clot lysis time does not depend on the amount of the added PAs. That is, in the case where the treated plasma clot lysis time and the untreated plasma clot lysis time are the same time, it is shown that the fibrinolytic resistance activity by PAI-1 and α2AP in blood plasma is lost by the fibrinolytic activity due to the addition of PAs to blood plasma. Therefore, in the first lysis time measurement step and the second lysis time measurement step, the treated plasma clot lysis time and the untreated plasma clot lysis time are measured at various PAs concentrations to evaluate the fibrinolytic resistance activity by PAI-1 and α2AP in the blood plasma of the test animal based on the PAs concentration in the case where the time difference between the treated plasma clot lysis time and the untreated plasma clot lysis time is sufficiently small, preferably within 10 minutes. It can be evaluated that the higher the concentration of PAs in the case where the time difference between the treated plasma clot lysis time and the untreated plasma clot lysis time is within 10 minutes is, the higher the fibrinolytic resistance activity by PAI-1 and α2AP in the blood plasma is, and the lower the concentration of PAs in the case where the time difference is within 10 minutes is, the lower the fibrinolytic resistance activity by PAI-1 and α2AP in the blood plasma is.

In the case where the fibrinolytic resistance activity is evaluated based on the concentration of PAs at which the time difference between the treated plasma clot lysis time and the untreated plasma clot lysis time is sufficiently small, the formation of the plasma clot in the first lysis time measurement step and the second lysis time measurement step may be performed in the presence of PAs at two or more different concentrations, and the concentrations of the PAs are not particularly limited. Since the concentration of PAs in which the time difference between the treated plasma clot lysis time and the untreated plasma clot lysis time is sufficiently small can be examined with a small number of test areas, in the present invention, the concentration of PAs is preferably set to a concentration of a 2-fold dilution series in a range of 0.1 to 10 nM.

In the case where the plasma clot lysis time is used as the index value of the fibrinolytic resistance activity, the method of evaluating fibrinolytic resistance activity in blood plasma according to the embodiment of the present invention can be very easily performed by sequentially adding an anionic surfactant, a nonionic surfactant and PAs, and thrombin to blood plasma at room temperature to 37°C and visually determining the presence or absence of lysis of the formed plasma clot. Therefore, by using the method of evaluating fibrinolytic resistance activity in blood plasma according to the embodiment of the present invention, the fibrinolytic resistance activity by PAI-1 and α2AP specific to each blood plasma, can be easily evaluated.

### <Case where plasmin generation rate is used as index value of fibrinolytic resistance activity by PAI-1 and α2AP>

In the case where the plasmin generation rate is used as an index value of the fibrinolytic resistance activity by PAI-1 and α2AP, the first fibrinolytic resistance measurement step is the following first plasmin generation rate measurement step, and the second fibrinolytic resistance measurement step is the following second plasmin generation rate measurement step. Therefore, the evaluation step is the following evaluation step.

A first plasmin generation rate measurement step of adding a nonionic surfactant, PAs, and a plasmin fluorescent substrate to the treated blood plasma obtained by the pretreatment step, further adding thrombin thereto to form a plasma clot, and then measuring a plasmin generation rate from the time at which the thrombin is added.

A second plasmin generation rate measurement step of adding a nonionic surfactant, PAs, and a plasmin fluorescent substrate to the part of the blood plasma derived from the test animal, further adding thrombin thereto to form a plasma clot, and then measuring a plasmin generation rate from the time at which the thrombin is added.

An evaluation step of evaluating the fibrinolytic resistance activity by PAI-1 and α2AP in the blood plasma of the test animal based on a ratio of the plasmin generation rate in the treated blood plasma measured in the first plasmin generation rate measurement step to the plasmin generation rate in the untreated blood plasma measured in the second plasmin generation rate measurement step.

In a first plasmin generation rate measurement step, a nonionic surfactant, PAs, and a plasmin fluorescent substrate are added to the treated blood plasma obtained by the pretreatment step, then thrombin is further added thereto to form a plasma clot, and then the plasma clot is lysed. The plasmin generation rate of the treated blood plasma after the addition of thrombin is measured. The order of addition of the nonionic surfactant, PAs, and the plasmin fluorescent substrate to the treated blood plasma is not particularly limited, and any of them may be added first, or both may be added at the same time. By adding the nonionic surfactant before the addition of thrombin, the plasmin generation in the case of the PAs-dependent lysis of the plasma clot formed in the treated blood plasma can be performed in the presence of the nonionic surfactant.

As the nonionic surfactant added to the treated blood plasma in the first plasmin generation rate measurement step, the nonionic surfactant listed as the nonionic surfactant used in the first lysis time measurement step can be used in the same manner. Also as the nonionic surfactant used in the first plasmin generation rate measurement step, polyoxyethylene alkylphenyl ether is preferable, and Triton X-100, which is widely used as an additive for cosmetics and pharmaceuticals, is particularly preferable from the viewpoint of having a sufficient effect.

The amount of the nonionic surfactant added to the treated blood plasma in the first plasmin generation rate measurement step may be any amount that is necessary and sufficient to suppress the activities of PAI-1 and α2AP that may be contained in the treated blood plasma, and can be appropriately determined in consideration of the type of the nonionic surfactant used and the like. The concentration of the nonionic surfactant added to the treated blood plasma is preferably within a concentration range in which the treated plasma clot lysis time (T¹) is constant, and is also preferably a relatively low within the concentration range such that the influence on the biological components other than PAI-1 and α2AP is not excessive. For example, in the case of Triton X-100, it is preferable to add Triton X-100 such that the concentration of Triton X-100 in the treated blood plasma to which thrombin has been added is about 1.0 v/v%.

As the PAs added to the treated blood plasma in the first plasmin generation rate measurement step, the PAs listed as the PAs used in the first lysis time measurement step can be used in the same manner. In addition, the amount of PAs added to the treated blood plasma in the first plasmin generation rate measurement step is not particularly limited, and can be, for example, 0.1 to 10 nM, preferably 0.5 to 10 nM, and more preferably 1.0 to 5.0 nM.

The plasmin fluorescent substrate added to the treated blood plasma in the first plasmin generation rate measurement step is not particularly limited as long as it is a substrate that is cleaved by plasmin to emit fluorescence. As the plasmin fluorescent substrate, for example, a fluorescent synthetic substrate in which a fluorescent substance is bonded to the C-terminus of a tripeptide consisting of Glu-Lys-Lys or a tripeptide consisting of Val-Leu-Lys, and the like can be used. As the fluorescent synthetic substrate cleaved by such plasmin, those included in a commercially available plasmin activity kit and the like can also be appropriately used. The amount of the plasmin fluorescent substrate added to the treated blood plasma in the first plasmin generation rate measurement step is not particularly limited, and can be, for example, 50 to 1,000 µM, and preferably 100 to 800 µM.

In the first plasmin generation rate measurement step, as the thrombin added to the treated blood plasma to which the nonionic surfactant, PAs, and the plasmin fluorescent substrate have been added, the thrombin listed as the thrombin used in the first lysis time measurement step can be used in the same manner. In addition, the amount of thrombin added to the treated blood plasma in the first plasmin generation rate measurement step is not particularly limited, and can be, for example, 0.1 to 10 units, and preferably 0.5 to 5 units.

In the case where the thrombin is added to the treated blood plasma to which the nonionic surfactant, PAs, and the plasmin fluorescent substrate have been added, plasma clots are created. The plasmin generated by PAs also degrades the added plasmin fluorescent substrate in addition to the created plasma clot. The higher the plasmin activity in the treated blood plasma is, that is, the larger the amount of plasmin generated by the added PAs is, the higher the fluorescence brightness value of the treated blood plasma. Therefore, by using the plasmin fluorescent substrate, it is possible to evaluate the enhancement of plasmin activity associated with the formation and lysis of plasma clots using the plasmin generation rate as an index.

The plasmin generation rate of the treated blood plasma measured in the first plasmin generation rate measurement step is a value obtained by dividing the amount of change in the fluorescence brightness value of the treated blood plasma at any point of time (T¹_{A}) from the point of time (T¹_{S}) at which the thrombin is added to the treated blood plasma to which the nonionic surfactant, the PAs, and the plasmin fluorescent substrate have been added, by the square of the elapsed time from the point of time (T¹_{S}) at which the thrombin is added to the point of time at which the fluorescence brightness value is measured. Specifically, the plasmin generation rate of the treated blood plasma is a value ([plasmin generation rate of treated blood plasma] = [([F_{A}] - [F¹_{S}])/([T¹_{A} (minutes)] - [T¹_{S} (minutes)])²) (dF/minutes² = dF/min²) obtained by dividing the difference ([F¹_{A}] - [F¹_{S}]) between the fluorescence brightness value (F¹_{A}) of the treated blood plasma at any subsequent point of time (T¹_{A}) and the fluorescence brightness value (F¹_{S}) of the treated blood plasma at the point of time (T¹_{S}) at which the thrombin is added to the treated blood plasma to which the nonionic surfactant, the PAs, and the plasmin fluorescent substrate have been added, by the square of the time ([T¹ (minutes)] = [T¹_{A} (minutes)] - [T¹_{S} (minutes)]) from the point of time (T¹_{S}) at which the thrombin is added to the point of time (T¹_{A}) at which the fluorescence brightness value of the treated blood plasma is measured.

In the case where the fluorescence brightness value of the treated blood plasma to which the nonionic surfactant, the PAs, the plasmin fluorescent substrate, and the thrombin have been added is measured over time, the plasmin generation rate at the point of time at which each fluorescence brightness value is measured based on the obtained measurement data is calculated, and the plasmin generation rate ([F¹_{A}] - [F¹_{S}])/([T¹_{A} (minutes)] - [T¹_{S} (minutes)])²) (dF/min²) as the vertical axis is plotted to the elapsed time (T¹_{A} (minutes)) from the addition of the thrombin as the horizontal axis, the plot is approximated to a straight line. The inclination of the approximate straight line corresponds to the plasmin generation rate. Therefore, the fluorescence brightness value of the treated blood plasma may be measured over time from the point of time at which the thrombin is added, or may be measured only at two points in time, which are the point of time at which the thrombin is added and any point of time after that. The point of time (T¹_{S}) at which the fluorescence brightness value of the treated blood plasma is measured to calculate the plasmin generation rate is not particularly limited as long as it is after the addition of the thrombin to the treated blood plasma, and for example, is preferably any point of time until 300 minutes after the addition of the thrombin, more preferably any point of time until 180 minutes after the addition of the thrombin, still more preferably any point of time until 120 minutes after the addition of the thrombin, and even still more preferably any point of time until 60 minutes after the addition of the thrombin. In particular, the point of time (T¹_{S}) at which the fluorescence brightness value of the treated blood plasma is measured is preferably any point of time from 1 minute to 60 minutes and more preferably any point of time from 10 minutes to 60 minutes after the addition of the thrombin to the treated blood plasma.

Among the blood plasma derived from the test animal, the residual portion or some of the residual portion which is not used in the pretreatment step (untreated blood plasma) is used in the second plasmin generation rate measurement step. The second plasmin generation rate measurement step is performed in the same manner as the first plasmin generation rate measurement step except that untreated blood plasma is used instead of the treated blood plasma. That is, in the second plasmin generation rate measurement step, the same type of nonionic surfactant, PAs, plasmin fluorescent substrate, and thrombin as those added to the treated blood plasma in the first plasmin generation rate measurement step are used. Furthermore, the concentrations of the nonionic surfactant, PAs, plasmin fluorescent substrate, and thrombin added to the untreated blood plasma are equal to the concentrations of the nonionic surfactant, PAs, and thrombin added to the treated blood plasma in the first plasmin generation rate measurement step.

Specifically, the plasmin generation rate of the untreated blood plasma measured in the second plasmin generation rate measurement step is a value ([plasmin generation rate of untreated blood plasma] = ([F²_{A}] - [F²_{S}])/([T²_{A} (minutes)] - [T²_{S} (minutes)])²) (dF/min²) obtained by dividing the amount of change ([F²_{A}] - [F²_{S}]) in the fluorescence brightness value of the untreated blood plasma at any point of time (T²_{A}) from the point of time (T²_{S}) at which the thrombin is added to the untreated blood plasma to which the nonionic surfactant, the PAs, and the plasmin fluorescent substrate have been added (F²_{S}: fluorescence brightness value of untreated blood plasma at point of time at which thrombin is added, F²_{A}: fluorescence brightness value of untreated blood plasma at T²_{A}), by the square of the elapsed time (T² = [T²_{A} (min)] - [T²_{S} (min)]) from the point of time (T²_{S}) at which the thrombin is added to the point of time at which the fluorescence brightness value is measured. The point of time (T²_{S}) at which the fluorescence brightness value of the untreated blood plasma is measured to calculate the plasmin generation rate is not particularly limited as long as it is after the addition of the thrombin to the untreated blood plasma, and for example, is preferably any point of time until 300 minutes after the addition of the thrombin, more preferably any point of time until 180 minutes after the addition of the thrombin, still more preferably any point of time until 120 minutes after the addition of the thrombin, and even still more preferably any point of time until 60 minutes after the addition of the thrombin. In particular, the point of time (T²_{S}) at which the fluorescence brightness value of the untreated blood plasma is measured is preferably any point of time from 1 minute to 60 minutes and more preferably any point of time from 10 minutes to 60 minutes after the addition of the thrombin to the untreated blood plasma. In addition, the point of time (T²_{S}) at which the fluorescence brightness value of untreated blood plasma is measured to calculate the plasmin generation rate may be the same as or different from the point of time (T¹_{S}) at which the fluorescence brightness value of treated blood plasma is measured to calculate the plasmin generation rate.

The ratio ([plasmin generation rate of treated blood plasma]/[plasmin generation rate of untreated blood plasma]) of the plasmin generation rate of the treated blood plasma measured in the first plasmin generation rate measurement step to the plasmin generation rate of the untreated blood plasma measured in the second plasmin generation rate measurement step is dependent on the fibrinolytic resistance by PAI-1 and α2AP in the blood plasma. Therefore, in the evaluation step, the fibrinolytic resistance activity by PAI-1 and α2AP in the blood plasma of the test animal is evaluated based on the ratio of the plasmin generation rate of the treated blood plasma to the plasmin generation rate of the untreated blood plasma. It can be evaluated that the larger the ratio between the plasmin generation rates of both blood plasma is, the higher the fibrinolytic resistance activity by PAI-1 and α2AP in the blood plasma of the test animal is, and the smaller the ratio between the plasmin generation rates of both blood plasma is, the lower the fibrinolytic resistance activity by PAI-1 and α2AP in the blood plasma of the test animal is.

The fibrinolytic resistance of blood plasma is basically determined by the quality and quantity of PAI-1 and α2AP, which are the major physiological inhibitors of the fibrinolytic system. Therefore, the amount of PAs administered in the thrombolytic therapy needs to be a sufficient amount to suppress the fibrinolytic resistance by PAI-1 and α2AP in the blood plasma and to enable the rapid plasmin generation by PAs. In addition, in the anti-fibrinolytic therapy, it is important to grasp the fibrinolytic resistance activity by PAI-1 and α2AP in the blood plasma of the patient as accurately as possible for determining a propriety of the anti-fibrinolytic therapy can be performed and for determining the dose of the anti-fibrinolytic drug such as tranexamic acid. By administering tranexamic acid after understanding the hyperfibrinolytic pathophysiology of the patient, it is possible to avoid the onset of thrombosis due to fibrin lysis failure. The method of evaluating fibrinolytic resistance activity in blood plasma according to the embodiment of the present invention is very useful for examining the fibrinolytic resistance of patients to whom thrombolytic therapy or anti-fibrinolytic therapy is applied, since the fibrinolytic resistance by PAI-1 and α2AP in blood plasma can be relatively easily evaluated.

The method of evaluating fibrinolytic resistance activity in blood plasma according to the embodiment of the present invention is extremely suitable for evaluating the fibrinolytic resistance activity by PAI-1 and α2AP in blood plasma collected from a test animal in which the concentration of a fibrinolysis factor in blood is significantly deviated from the normal value. By performing the method of evaluating fibrinolytic resistance activity in blood plasma according to the embodiment of the present invention on blood plasma derived from a patient on which the thrombolytic therapy is performed, it is possible to set a PAs dose that is safe and from which sufficient thrombolysis can be expected, or to obtain important information for the setting. In addition, by performing the method of evaluating fibrinolytic resistance activity in blood plasma according to the embodiment of the present invention on blood plasma derived from a patient on whom the anti-fibrinolytic therapy is performed, it is possible to appropriately determine a propriety of tranexamic acid administration, or to obtain important information for the administration. In particular, the method of evaluating fibrinolytic resistance activity in blood plasma according to the embodiment of the present invention can be easily performed at the bedside of a patient or in a general examination room. Therefore, in the case of performing thrombolytic therapy or anti-fibrinolytic therapy, the fibrinolytic resistance activity by PAI-1 and α2AP specific to the blood plasma of the patient can be rapidly and easily evaluated at the bedside of the patient or the like.

The method of evaluating fibrinolytic resistance activity in blood plasma according to the embodiment of the present invention is performed on blood plasma derived from a patient on which thrombolytic therapy is performed, and based on the obtained evaluation result, the presence or absence of the necessity of thrombolytic therapy and the dose of PAs in the thrombolytic therapy are determined. For example, in the case where the evaluation is obtained that the fibrinolytic resistance activity by PAI-1 and α2AP in the blood plasma of a patient is higher than the fibrinolytic resistance activity by PAI-1 and α2AP in the blood plasma of a healthy subject, the PAs dose to the patient is determined to be larger than the dose recommended in a standard protocol for thrombolytic therapy. In addition, in the case where the evaluation is obtained that the fibrinolytic resistance activity by PAI-1 and α2AP in the blood plasma of a patient is lower than the fibrinolytic resistance activity by PAI-1 and α2AP in the blood plasma of a healthy subject, the PAs dose to the patient is determined to be smaller than the dose recommended in a standard protocol for thrombolytic therapy. In addition, in the case where the fibrinolytic resistance activity by PAI-1 and α2AP in the blood plasma of a patient is evaluated based on the concentration of PAs, the PAs dose to the patient can be set to an amount required to make the concentration of PAs in the blood plasma equal to the concentration of PAs at which the time difference between the treated plasma clot lysis time and the untreated plasma clot lysis time is sufficiently small. In addition, in the case where the time difference between the treated plasma clot lysis time and the untreated plasma clot lysis time at the time of administration of a low concentration of PAs is sufficiently large, it can be evaluated that the PAs dose to the patient is not an excessive amount.

The method of evaluating fibrinolytic resistance activity in blood plasma according to the embodiment of the present invention is performed on blood plasma derived from a patient on whom the anti-fibrinolytic therapy is performed, and based on the obtained evaluation result, the presence or absence of the necessity of the anti-fibrinolytic therapy and the propriety of the tranexamic acid administration in the anti-fibrinolytic therapy are determined. For example, in the case where the evaluation is obtained that the fibrinolytic resistance activity by PAI-1 and α2AP in the blood plasma of a patient is hardly recognized, it can be determined that an anti-fibrinolytic therapy by tranexamic acid administration to the patient is necessary. In addition, in the case where the evaluation is obtained that the fibrinolytic resistance activity by PAI-1 and α2AP in the blood plasma of a patient is equal to or higher than the fibrinolytic resistance activity by PAI-1 and α2AP in the blood plasma of a healthy subject, it can be determined that the patient is not suitable for the anti-fibrinolytic therapy.

The method of evaluating fibrinolytic resistance activity in blood plasma according to the embodiment of the present invention can be used in combination with a measurement method of fibrinolytic resistance activity or fibrinolytic activity of coagulation and fibrinolytic system based on other indexes. For example, the method of evaluating fibrinolytic resistance activity in blood plasma according to the embodiment of the present invention can be used in combination with TEG or ROTEM using a change in viscosity of an existing blood clot as an index.

### [Examples]

Hereinafter, the present invention will be described in more detail with reference to examples, but the present invention is not limited to the following examples.

In the tPA used in the following Examples, tPA provided from TOYOBO CO., LTD. and DAIICHI PHARMACEUTICAL CO., LTD., and tPA manufactured by Tanabe Mitsubishi Pharma Corporation were used as a human genetic recombination single-strand tPA. As the plasmin fluorescent substrate, t-Butyloxycarbonyl-L-glutamyl-L-lysyl-L-lysine 4-methylcoumaryl-7-amide (Boc-Glu-Lys-Lys-MCA: manufactured by Peptide Institute, Inc.) was used. Human thrombin manufactured by Sigma-Aldrich Co., LLC was used.

### [Example 1]

The fibrinolytic resistance activities by PAI-1 and α2AP in blood plasma derived from a healthy subject (normal blood plasma) and α2AP-deficient blood plasma were evaluated.

Normal blood plasma was collected by drawing blood from a healthy person into a blood collection tube containing an anticoagulant and performing a centrifugation treatment to recover the blood plasma.

As the α2AP-deficient blood plasma, a commercially available product (manufactured by Affinity Biologicals, Inc.) was used.

SDS was added to a half amount of each blood plasma such that a final concentration is 0.1 w/v%, and the mixture was incubated at room temperature for 10 minutes to perform SDS treatment. Thereafter, 1.0 v/v% of Triton X-100 and 0.5, 1.0, 2.0, or 4.0 nM of tPA were added to the obtained treated blood plasma, then human thrombin was added thereto such that a final concentration is 1.0 unit/mL to cause to form a plasma clot, and the treated plasma clot lysis time was measured. The presence or absence of lysis of the plasma clot was visually determined.

In addition, Triton X-100 and tPA were added to the SDS-untreated blood plasma in the same manner, then human thrombin was added thereto, and the untreated plasma clot lysis time was measured.

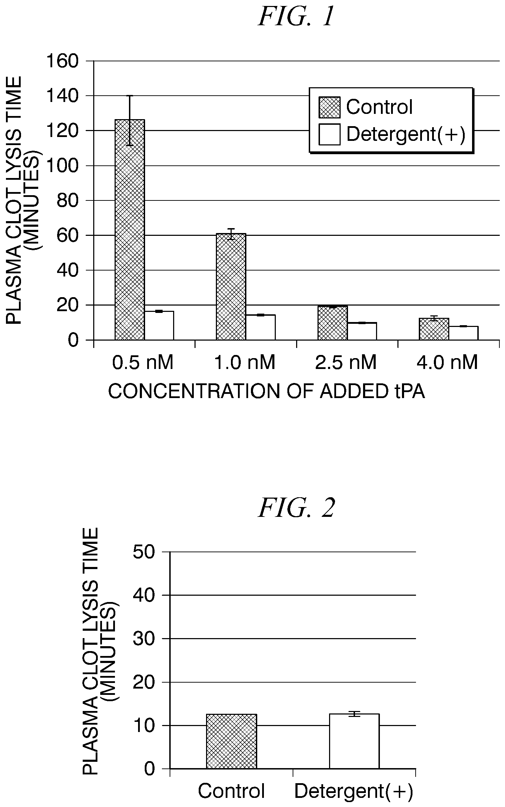

The measurement results of the SDS-treated plasma clot lysis time (minutes) and the untreated plasma clot lysis time (minutes) of the normal blood plasma are shown in FIG. 1 and Table 1, and the measurement results of the treated plasma clot lysis time (minutes) and the untreated plasma clot lysis time (minutes) of the α2AP-deficient blood plasma are shown in FIG. 2 and Table 1.

**[Table 1]**

| | Clot lysis time (minutes) | | | |
|---|---|---|---|---|
| | tPA 0.5 nM | tPA 1.0 nM | tPA 2.0 nM | tPA 4.0 nM |
| Untreated: Control | 126 ± 14.5 | 61 ± 2.1 | 19 ± 0.5 | 12 ± 0.5 |
| Treated: Detergent (+) | 16 ± 0.5 | 13 ± 0.5 | 10 ± 0 | 8 ± 0.5 |

As shown in FIG. 1, in the normal blood plasma, the SDS-untreated (control) plasma clot lysis time was shortened depending on the tPA concentration, but the plasma clot lysis time of the blood plasma after the SDS treatment is short even at a low tPA concentration, and the shortening depending on the tPA concentration was mild. On the other hand, as shown in FIG. 2, in the case where the α2AP-deficient blood plasma was used, the plasma clot lysis time was short, and the plasma clot lysis time was not affected by the SDS treatment.

### [Example 2]

The fibrinolytic resistance activities by PAI-1 and α2AP in blood plasma derived from a healthy subject (normal blood plasma) and α2AP-deficient blood plasma were evaluated in the same manner as in Example 1.

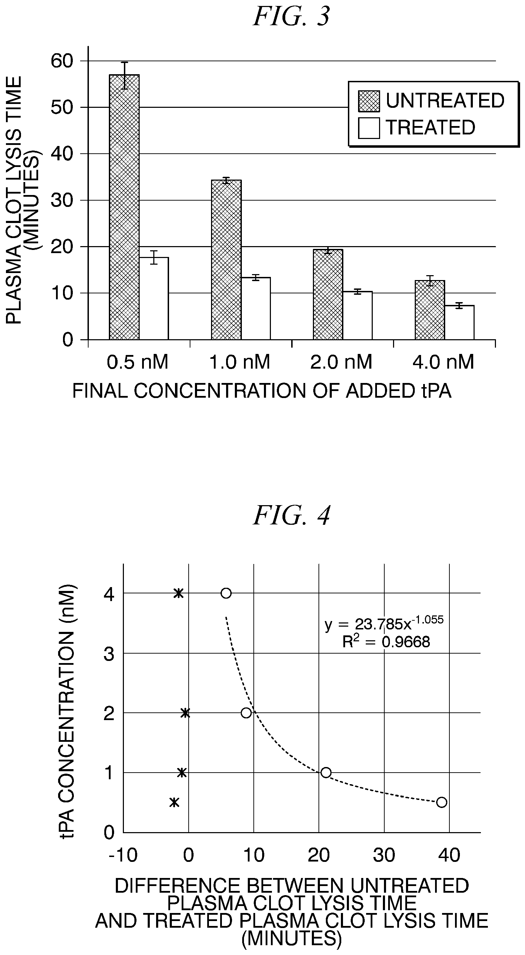

The measurement results of the SDS-treated plasma clot lysis time (minutes) and the untreated plasma clot lysis time (minutes) of normal blood plasma are shown in FIG. 3. In addition, the plot of the correlation (o) between the difference (minutes) between the SDS-treated plasma clot lysis time and the untreated plasma clot lysis time in the normal blood plasma and the tPA concentration (nM) and the plot of the correlation (*) between the difference (minutes) between the SDS-treated plasma clot lysis time and the untreated plasma clot lysis time in α2AP-deficient blood plasma and the tPA concentration (nM) were shown in FIG. 4.

As shown in FIG. 3, in the normal blood plasma, the SDS-untreated plasma clot lysis time was shortened depending on the tPA concentration, but the plasma clot lysis time of the blood plasma after the SDS treatment is short even at a low tPA concentration, and the shortening depending on the tPA concentration was mild. In addition, as shown in FIG. 4, in the case where normal blood plasma was used, the difference (minutes) between the SDS-treated plasma clot lysis time and the untreated plasma clot lysis time, and the tPA concentration (nM) were approximated by a power curve, but in the case where the α2AP-deficient blood plasma was used, there was no difference between the untreated plasma clot lysis time and treated plasma clot lysis time.

### [Example 3]

The fibrinolytic resistance activities by PAI-1 and α2AP in blood plasma derived from a healthy subject (normal blood plasma) and α2AP-deficient blood plasma were evaluated as a plasmin generation rate.

Normal blood plasma was collected by drawing blood from a healthy person into a blood collection tube containing an anticoagulant and performing a centrifugation treatment to recover the blood plasma.

As the α2AP-deficient blood plasma, a commercially available product (manufactured by Affinity Biologicals, Inc.) was used.

SDS was added to a half amount of each blood plasma such that a final concentration is 0.1 w/v%, and the mixture was incubated at room temperature for 10 minutes to perform SDS treatment. Thereafter, 1.0 v/v% of Triton X-100, 0.5, 1.0, 2.0, 4.0, 8.0, or 16.0 nM of tPA, and 100 µM of a plasmin fluorescent substrate were added to the obtained treated blood plasma, then human thrombin was added thereto such that a final concentration is 1.0 unit/mL to cause to form a plasma clot, and then the plasma clot was lysed. The fluorescence brightness value of the treated blood plasma was measured over time after the addition of the thrombin. Next, using spreadsheet software (linear approximation of "Microsoft Excel"), the plasmin generation rate of the treated blood plasma (amount of change in fluorescence brightness value from point of time at which thrombin is added/square of elapsed time (minutes) from point of time at which thrombin is added to point of time at which fluorescence brightness value was measured: dF/min²) was measured from the obtained measurement values.

In addition, in the same manner, Triton X-100, tPA, and a plasmin fluorescent substrate were also added to the SDS-untreated blood plasma, then human thrombin was added thereto, the fluorescence brightness value of the treated blood plasma was measured over time after the addition of the thrombin. From the obtained measurement value, the plasmin generation rate of the untreated blood plasma (amount of change in fluorescence brightness value from point of time at which thrombin is added/square of elapsed time (minutes) from point of time at which thrombin is added to point of time at which fluorescence brightness value was measured: dF/min²) was measured.

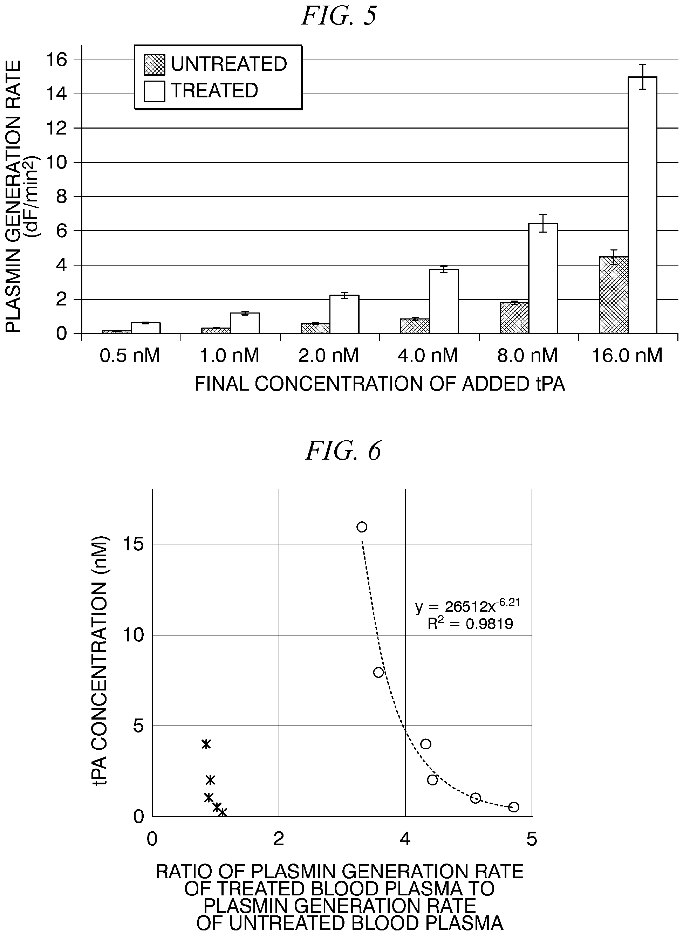

The measurement results of the plasmin generation rate (dF/min²) of the treated blood plasma and the plasmin generation rate (dF/min²) of the untreated blood plasma in normal blood plasma are shown in FIG. 5. In addition, the plot of the correlation (o) between the ratio of the plasmin generation rate of the treated blood plasma to the plasmin generation rate of the untreated blood plasma in normal blood plasma and the tPA concentration and the plot of the correlation (*) between the ratio of the plasmin generation rate of the treated blood plasma to the plasmin generation rate of the untreated blood plasma in α2AP-deficient blood plasma and a tPA concentration (nM) were shown in FIG. 6.

As shown in FIG. 5, in normal blood plasma, the plasmin generation rate was increased by SDS treatment in a tPA concentration-dependent manner as compared with the case where SDS treatment were not performed. In addition, as shown in FIG. 6, in the case where normal blood plasma was used, the ratio of the plasmin generation rate of the treated blood plasma to the plasmin generation rate of the untreated blood plasma, and the tPA concentration (nM) was approximated by a power curve, but in the case where α2AP-deficient blood plasma was used, such a relationship was not recognized.

### [Industrial Applicability]

By using the method of evaluating fibrinolytic resistance activity in blood plasma according to the embodiment of the present invention, the fibrinolytic resistance activity by PAI-1 and α2AP specific to blood plasma can be easily evaluated. The evaluation method is particularly useful for setting a safe and efficient dose of PAs in thrombolytic therapy, since the fibrinolytic resistance activity by PAI-1 and α2AP can be evaluated even for blood plasma in which the concentration of a fibrinolysis factor in blood is out of the normal range, such as sepsis or COVID-19.

## Claims

1. A method of evaluating fibrinolytic resistance activity in blood plasma of a test animal, the method comprising:
a pretreatment step of adding an anionic surfactant to some of a blood plasma sample derived from the test animal and incubating the blood plasma sample for a predetermined time;
a first fibrinolytic resistance measurement step of measuring an index value of fibrinolytic resistance activity by PAI-1 and α2AP in the treated blood plasma sample obtained by the pretreatment step;
a second fibrinolytic resistance measurement step of measuring an index value of fibrinolytic resistance activity by PAI-1 and α2AP in some of the blood plasma sample derived from the test animal; and
an evaluation step of evaluating the fibrinolytic resistance activity by PAI-1 and α2AP in the blood plasma of the test animal, based on the index value measured in the first fibrinolytic resistance measurement step and the index value of untreated blood plasma measured in the second fibrinolytic resistance measurement step,
wherein the index value is the time until a plasma clot formed by thrombin in the presence of a nonionic surfactant and a plasminogen activator is lysed, or a plasmin generation rate in the case of adding thrombin in the presence of a nonionic surfactant and a plasminogen activator.

2. The method of evaluating fibrinolytic resistance activity in blood plasma according to Claim 1,
wherein the anionic surfactant is SDS.

3. The method of evaluating fibrinolytic resistance activity in blood plasma according to Claim 2,
wherein the pretreatment step is performed by adding 0.01 to 0.1 w/v% of SDS to some of the blood plasma sample and treating the blood plasma sample for 5 to 30 minutes.

4. The method of evaluating fibrinolytic resistance activity in blood plasma according to Claim 1,
wherein the nonionic surfactant is Triton X-100.

5. The method of evaluating fibrinolytic resistance activity in blood plasma according to Claim 1,
wherein the index value is the time until the plasma clot formed by thrombin in the presence of the nonionic surfactant and the plasminogen activator is lysed,
the first fibrinolytic resistance measurement step, the second fibrinolytic resistance measurement step, and the evaluation step are each
a first lysis time measurement step of adding a nonionic surfactant and a plasminogen activator to the treated blood plasma sample obtained in the pretreatment step, further adding thrombin thereto to form a plasma clot, and then measuring the time until the formed plasma clot is lysed from the time at which the thrombin is added,
a second lysis time measurement step of adding a nonionic surfactant and a plasminogen activator to some of the blood plasma sample derived from the test animal, further adding thrombin thereto to form a plasma clot, and then measuring the time until the formed plasma clot is lysed from the time at which the thrombin is added, and
an evaluation step of evaluating the fibrinolytic resistance activity by PAI-1 and α2AP in the blood plasma of the test animal based on a difference between the lysis time of the plasma clot of the treated blood plasma measured in the first lysis time measurement step and the lysis time of the plasma clot of the untreated blood plasma measured in the second lysis time measurement step, and
concentrations of the nonionic surfactant, the plasminogen activator, and the thrombin, which are added to the untreated blood plasma in the second lysis time measurement step, are equal to concentrations of the nonionic surfactant, the plasminogen activator, and the thrombin, which are added to the treated blood plasma in the first lysis time measurement step.

6. The method of evaluating fibrinolytic resistance activity in blood plasma according to Claim 5,
wherein the nonionic surfactant is Triton X-100, and
the formation of the plasma clot in the first lysis time measurement step and the second lysis time measurement step is performed in the presence of 1.0 v/v% Triton X-100.

7. The method of evaluating fibrinolytic resistance activity in blood plasma according to Claim 5,
wherein the formation of the plasma clot in the first lysis time measurement step and the second lysis time measurement step is performed in the presence of the plasminogen activators at two or more different concentrations, and
in the evaluation step, the fibrinolytic resistance activity by PAI-1 and α2AP in the blood plasma of the test animal is evaluated based on a concentration of the plasminogen activator in the case where a difference between the lysis time of the plasma clot of the treated blood plasma measured in the first lysis time measurement step and the lysis time of the plasma clot of the untreated blood plasma measured in the second lysis time measurement step is within 10 minutes.

8. The method of evaluating fibrinolytic resistance activity in blood plasma according to Claim 1,
wherein the index value is the plasmin generation rate in the case of adding thrombin in the presence of the nonionic surfactant and the plasminogen activator,
the first fibrinolytic resistance measurement step, the second fibrinolytic resistance measurement step, and the evaluation step are each
a first plasmin generation rate measurement step of adding a nonionic surfactant, a plasminogen activator, and a plasmin fluorescent substrate to the treated blood plasma obtained by the pretreatment step, further adding thrombin thereto to form a plasma clot, and then measuring a plasmin generation rate from the time at which the thrombin is added,
a second plasmin generation rate measurement step of adding a nonionic surfactant, a plasminogen activator, and a plasmin fluorescent substrate to some of the blood plasma sample derived from the test animal, further adding thrombin thereto to form a plasma clot, and then measuring a plasmin generation rate from the time at which the thrombin is added, and
an evaluation step of evaluating the fibrinolytic resistance activity by PAI-1 and α2AP in the blood plasma of the test animal based on a ratio of the plasmin generation rate in the treated blood plasma measured in the first plasmin generation rate measurement step to the plasmin generation rate in the untreated blood plasma measured in the second plasmin generation rate measurement step, and
concentrations of the nonionic surfactant, the plasminogen activator, the plasmin fluorescent substrate, and thrombin, which are added to the untreated blood plasma in the second plasmin generation rate measurement step, are equal concentrations of the nonionic surfactant, the plasminogen activator, the plasmin fluorescent substrate, and the thrombin, which are added to the treated blood plasma in the first plasmin generation rate measurement step.

9. The method of evaluating fibrinolytic resistance activity in blood plasma according to Claim 8,
wherein the plasmin generation rate is a value obtained by dividing an amount of change in a fluorescence brightness value from the point of time at which the thrombin is added by a square of an elapsed time from the point of time at which the thrombin is added.

10. The method of evaluating fibrinolytic resistance activity in blood plasma according to Claim 8,
wherein the nonionic surfactant is Triton X-100, and
the formation of the plasma clot in the first plasmin generation rate measurement step and the second plasmin generation rate measurement step is performed in the presence of 1.0 v/v% Triton X-100.

11. A method for determining a dose of a plasminogen activator in a thrombolytic therapy, the method comprising:
performing the method of evaluating fibrinolytic resistance activity in blood plasma according to any one of Claims 1 to 10 on a blood plasma sample derived from a patient on whom the thrombolytic therapy is performed; and
determining the dose of the plasminogen activator in the thrombolytic therapy based on an obtained evaluation result.

12. A method for determining a propriety of tranexamic acid administration in an anti-fibrinolytic therapy, the method comprising:
performing the method of evaluating fibrinolytic resistance activity in blood plasma according to any one of Claims 1 to 10 on a blood plasma sample derived from a patient on whom the anti-fibrinolytic therapy is performed; and
determining the propriety of tranexamic acid administration in the anti-fibrinolytic therapy based on an obtained evaluation result.
